(19) Europäisches Patentamt
European Patent Office
Office européen des brevets

(11) Veröffentlichungsnummer: **0 243 822 B1**

(12) # EUROPÄISCHE PATENTSCHRIFT

(45) Veröffentlichungstag der Patentschrift: **17.10.90**

(51) Int. Cl.⁵: **A61K 7/48**, C08B 37/08

(21) Anmeldenummer: **87105703.0**

(22) Anmeldetag: **16.04.87**

(54) **Kosmetische Mittel auf der Basis von N-Hydroxybutyl-chitosanen, neue N-Hydroxybutyl-chitosane sowie Verfahren zu ihrer Herstellung.**

(30) Priorität: **30.04.86 DE 3614697**

(43) Veröffentlichungstag der Anmeldung: **04.11.87 Patentblatt 87/45**

(45) Bekanntmachung des Hinweises auf die Patenterteilung: **17.10.90 Patentblatt 90/42**

(84) Benannte Vertragsstaaten: **AT BE CH DE ES FR GB GR IT LI NL SE**

(56) Entgegenhaltungen:
**EP-A- 0 028 126**
**EP-A- 0 193 736**

**DERWENT JAPANESE PATENT REPORT, Nr. 76-17481X, 1976; & JP-A-76 06 879**

**Die Akte enthält technische Angaben, die nach dem Eingang der Anmeldung eingereicht wurden und die nicht in dieser Patentschrift enthalten sind.**

(73) Patentinhaber: **Wella Aktiengesellschaft, Berliner Allee 65, D-6100 Darmstadt(DE)**

(72) Erfinder: **Lang, Günther, Dr., Auf der Roten Erde 10B, D-6107 Reinheim 5(DE)**
Erfinder: **Konrad, Eugen, Mecklenburger Strasse 101, D-6100 Darmstadt(DE)**
Erfinder: **Wendel, Harald, Grabengasse 3, D-6105 Ober-Ramstadt(DE)**
Erfinder: **Maresch, Gerhard, Hölgesstrasse 19, D-6100 Darmstadt(DE)**
Erfinder: **Lenz, Hans-Rudi, Moltkestrasse 16, D-6100 Darmstadt(DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

## Beschreibung

Die Erfindung betrifft kosmetische Mittel zur Behandlung von Haaren oder der Haut mit einem Gehalt an neuen makromolekularen, vom Chitosan abgeleiteten Verbindungen, welche in einer geeigneten Kosmetikgrundlage zur Anwendung kommen.

Die Erfindung betrifft weiterhin neue N-Hydroxybutyl-chitosane sowie Verfahren zu ihrer Herstellung.

Es ist bereits bekannt, in kosmetischen Mitteln, insbesondere für die Behandlung von Haaren, kationaktive Polymere, insbesondere Polymere, die quaternäre Ammoniumgruppen aufweisen, als Konditionierungsmittel einzusetzen. Auf Grund einer Wechselwirkung zwischen ihren Ammoniumgruppen und den anionischen Gruppen des Haares besitzen die katonaktiven Polymere eine große Affinität zur Keratinfaser.

Es wurde festgestellt, daß der Einsatz derartiger kationaktiver Polymere in solchen kosmetischen Mitteln zahlreiche Vortele ergibt: Die Entwirrung der Haare sowie deren Behandlung wird erleichtert, und weiterhin werden dem Haar Sprungkraft und Glanzwirkung veliehen. Durch die Affinität zum Keratin neigen diese Polymere jedoch bei wiederholter Anwendung zur Ansammlung auf den Haaren, so daß diese schwerer werden, was im Endeffekt unerwünscht ist.

Weiterhin ergeben sich bei synthetischen Polymeren Probleme wegen der physiologischen Wirkung von eventuell vorhandenen Monomerspuren, die nur schwer aus dem Polymer entfernbar sind.

Man hat bereits versucht, diese vorerwähnten Nachteile dadurch zu beheben, daß wasserlösliche Salze des Chitosans, eines durch Entacetylierung von Chitin herstellbaren Polyglucosamins, in derartigen kosmetischen Mitteln Anwendung finden. In diesem Zusammenhang wird Bezug genommen auf die eigene europäische Patentschrift 0 002 506 sowie die eigene deutsche Patentschrift 26 27 419.

In gleicher Weise wie bei der Mehrzahl der kationaktiven Polymere mit quaternärer Gruppierung ergibt Chitosan ebenfalls häufig den Nachteil, daß es mit den anionaktiven oberflächenaktiven Agenzien, die üblicherweise in kosmetischen Mitteln zur Behandlung von Haaren, insbesondere in Shampoos, Anwendung finden, wenig verträglich ist. Es ist daher notwendig, das Chitosan in separaten Behandlungen, nämlich vor und/oder nach der Shampoonierung zur Einwirkung zu bringen.

Das Chitosan erweist sich weiterhin in neutralem und alkalischem Medium als praktisch unlöslich, so daß seine Anwendung beispielsweise in alkalischen Dauerwellmitteln oder Haarfärbemitteln nicht möglich ist.

Durch Einsatz von Glycidylchitosanen gemäß unserer eigenen DE-OS 32 23 423 anstelle von Chitosansalzen lassen sich die vorstehend erwähnten Nachteile vermeiden. Die Umsetzung von Chitosan mit Glycid ist jedoch sehr kostenintensiv, da Glycid ein teurer, nicht großtechnisch hergestellter, Rohstoff ist.

Die vorstehend genannten Chitosane beziehungsweise Chitosanderivate weisen jedoch einen weiteren Nachteil auf, da sie nicht oder nur wenig in organischen Lösungsmitteln löslich sind. Deshalb wäre es von großem Vorteil, wenn solche Derivate in organischen Lösungsmitteln löslich sind, um dadurch die Einsatzmöglichkeiten in kosmetischen Mitteln zu erweitern.

Aufgabe der Erfindung ist es daher, kostengünstigere Mittel zur Verfügung zu stellen, mit denen sich die oben aufgeführten Nachteile vermeiden lassen.

Bei Fortführung der Untersuchungen mit Chitosan und den davon abgeleiteten Verbindungen wurde nunmehr gefunden, daß bestimmte Chitosan-Derivate, und zwar speziell N-Hydroxybutyl-chitosane, die vorstehend aufgeführten Nachteile ebenfalls nicht aufweisen und zudem wesentlich kostengünstiger als die bisher bekannten Glycidyl-chitosane erhältlich sind. Außerdem sind solche Derivate auch in organischen Lösungsmitteln, wie beispielsweise Ethanol und Isopropanol, löslich und ermöglichen so die Herstellung von wasserfreien, zum Beispiel alkoholischen, Lösungen.

Im Gegensatz zu synthetischen Polymeren mit endlichen Restmonomerengehalten sind diese N-Hydroxybutyl-chitosane physiologisch unbedenklich und biologisch abbaubar. Aufgrund ihrer Filmeigenschaften, ihrer Löslichkeit in organischen Lösungsmitteln, sowie ihrer Verdickerwirkung und Aniontensidverträglichkeit können sie nicht nur als neue, interessante Rohstoffe für Kosmetika, sondern ebenso in der Pharmazie, als Flockungs- und Verdickungsmittel in der Abwasseraufbereitung, als Appretur- und Schlichtemittel in der Textilindustrie sowie bei der Papierherstellung Anwendung finden.

Mit N-Hydroxybutyl-chitosanen oder ihren Salzen mit organischen oder anorganischen Säuren lassen sich somit kosmetische Mittel zur Behandlung von Haaren oder der Haut herstellen, die sich durch überraschend vorteilhafte Eigenschaften auszeichnen und dadurch gekennzeichnet sind, daß sie in einer geeigneten Kosmetikgrundlage ein N-Hydroxybutyl-chitosan, bestehend aus

a) 4 bis 40 Molprozent Monomereinheiten der Formel (I)

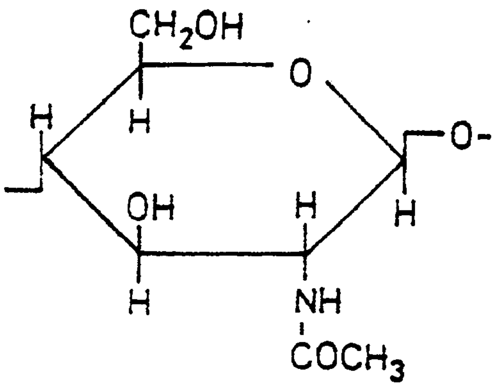

(I)

und

b) 60 bis 96 Molprozent Monomereinheiten der Formel (II)

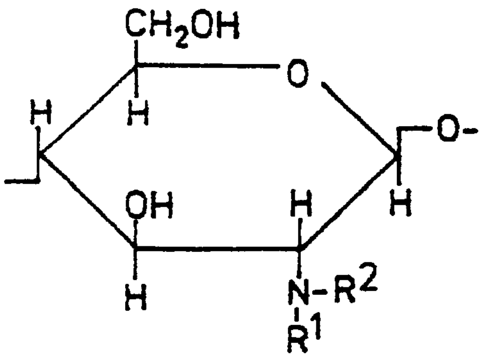

(II),

worin $R^1$ und $R^2$ gleich oder verschieden sind und Wasserstoff oder die Gruppe

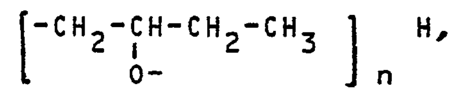

mit n gleich eine ganze Zahl von 1 bis 5, bedeuten, unter der Bedingung, daß bei mindestens 50 Prozent der Einheiten der Formel (II) $R^1$ und $R^2$ nicht gleichzeitig Wasserstoff darstellen, oder dessen lösliche Salze mit organischen oder anorganischen Säuren enthalten.

Die erfindungsgemäßen N-Hydroxybutyl-chitosan enthaltenden Mittel eignen sich ganz allgemein zur Behandlung der Haut und/oder der Haare. Sie können beispielsweise vorliegen als Haar- und/oder Körperwaschmittel, Tönungsshampoos, Frisiercremes, Frisierlotionen, Fönlotionen, Mittel zur Festigung der Frisur, Waschlotionen, Haarkuren, Mittel gegen Kopfschuppen, Mittel zur permanenten Haarverformung, Mittel zur Färbung oder Entfärbung von Haaren, Mittel zum Auftragen vor oder nach der Haarfärbung und als kosmetische Mittel zur Pflege, zum Schutz oder zur Reinigung der Haut wie Gesichtswässer, Rasierwässer, Feuchthaltecremes, Coldcremes, Körperlotionen, Sonnenschutzmittel oder auch Make-up-Präparate wie Schminkcremes und Rouges.

Der Gehalt der erfindungsgemäßen kosmetischen Mittel an N-Hydroxybutyl-chitosan liegt hierbei zweckmäßig bei 0,05 bis 10 Gewichtsprozent, vorzugsweise bei 0,05 bis 3,0 Gewichtsprozent.

Die kosmetischen Mittel gemäß der vorliegenden Erfindung können zusätzlich zu dem neuen Wirkstoff N-Hydroxybutyl-chitosan zur Herstellung einer Kosmetikgrundlage alle diejenigen Bestandteile enthalten, die in Haar- und Hautbehandlungsmitteln üblicherweise eingesetzt werden, insbesondere anionische, kationische, amphotere, zwitterionische oder nichtionische oberflächenaktive Tenside, Schaumsynergisten, Stabilisatoren, Sequestriermittel, Pigmente, Verdicker, Emulgatoren, Pufferstoffe, Konservierungsmittel, Farbstoffe, Parfümöle, bekannte kosmetische Polymere wie anionische, nichtionische, kationische oder amphotere Polymere, Naturstoffe, kosmetische Öle, Fettalkohole, Wachse, Schaumstabilisatoren, Wirkstoffe gegen Kopfschuppen, Reduktionsmittel und Treibgase.

Die erfindungsgemäßen kosmetischen Mittel weisen in bevorzugter Weise einen pH-Wert von 2 bis 11 auf und können in Form wäßriger, alkoholischer oder wäßrig-alkoholischer Zubereitungen, insbesondere als Lösungen, als Cremes, als Gele, als Dispersionen oder als Emulsionen vorliegen.

Ebenfalls ist es möglich, diese Mittel mit Hilfe eines Zerstäubers beziehungsweise anderer geeigneter

Sprühvorrichtungen oder im Gemisch mit üblichen unter Druck verflüssigten Treibmitteln als Aerosolspray (zum Beispiel Aerosolhaarspray) oder Aerosolschaum aus einem Druckbehälter zu entnehmen.

In bevorzugter Weise handelt es sich bei den erfindungsgemäßen kosmetischen Mitteln um Mittel zur Festigung der Frisur, wie flüssige Haarfestiger oder Haarsprays. Diese liegen üblicherweise als wäßrige, alkoholische oder wäßrig-alkoholische Lösungen vor, die durch einen Gehalt an N-Hydroxybutyl-chitosan bestehend aus Einheiten der vorstehend genannten Formeln (I) und (II) oder dessen löslichen Salzen mit organischen oder anorganischen Säuren gekennzeichnet sind. Hierbei kann das N-Hydroxybutyl-chitosan selbst als filmbildendes oder festigendes Harz eingesetzt werden; es können jedoch auch zusätzlich andere filmbildende natürliche oder synthetische kosmetische Polymere in dem erfindungsgemäßen Haarfestigungsmittel enthalten sein. Als natürliche Polymere kommen beispielsweise Schellack, Alginate, Gelatine, Pektine und Cellulosederivate in Betracht. Von den synthetischen Polymeren finden zum Beispiel Polyvinylpyrrolidon, Polyvinylacetat, Polyacrylverbindungen, wie beispielsweise Acrylsäure- oder Methacrylsäurepolymerisate, basische Polymerisate von Estern aus Acrylsäure oder Methacrylsäure mit Aminoalkoholen oder die Salze oder Quaternisierungsprodukte dieser basischen Polymerisate, Polyacrylnitril sowie Co- oder Terpolymerisate aus derartigen Verbindungen, beispielsweise Polyvinylpyrrolidon-Vinylacetat, Verwendung.

Die Mittel weisen dann insbesondere einen pH-Wert zwischen 6 und 8 auf. Solche Mittel zur Festigung der Frisur enthalten üblicherweise die filmbildenden Polymere in einer Gesamtmenge von etwa 0,05 bis 3,0 Gewichtsprozent. Enthalten die Mittel neben dem hier beschriebenen N-Hydroxybutyl-chitosan aus Einheiten der oben genannten Formeln (I) und (II) noch andere filmbildende Polymere, so reduziert sich der Gehalt an N-Hydroxybutyl-chitosan entsprechend.

Als Alkohole kommen insbesondere die für kosmetische Zwecke üblicherweise verwendeten niederen Alkohole mit 1 bis 4 Kohlenstoffatomen, wie zum Beispiel Ethanol und Isopropanol, in Betracht.

Wenn die Mittel zur Festigung der Frisur in Form von Aerosolpräparaten vorliegen, welche aus einem Druckbehälter versprüht werden, so enthalten sie in der Kosmetikgrundlage etwa 10 bis 60 Gewichtsprozent eines Treibmittels. Als Treibmittel können Chlorfluoralkane, wie zum Beispiel $CCl_3F$, $CCl_2F_2$, $C_2Cl_3F_3$, $(CCl_2F)_2$, $CHCl_2F$ und $(CClF_2)_2$, leichtflüchtige Kohlenwasserstoffe, wie zum Beispiel n-Butan und n-Propan, oder auch Dimethylether, Kohlendioxid, Distickstoffmonoxid, Stickstoff, Methylenchlorid und 1,1,1-Trichlorethan, Verwendung finden.

Die erfindungsgemäßen Mittel zur Festigung der Frisur können weiterhin die für solche Mittel üblichen Zusätze, wie beispielsweise Parfümöle, Bakterizide oder Fungizide, kämmbarkeitsverbessernde Substanzen und Modifiziermittel, wie zum Beispiel Siliconöl oder Weichmacher, wie beispelsweise Isopropylmyristat, Phthalsäurediethylester und Diethylstearat, enthalten.

Die erfindungsgemäßen Mittel zur Festigung der Frisur können gegebenenfalls durch einen Gehalt an kosmetischen Farbstoffen das Haar gleichzeitig färben oder tönen. Derartige Präparate sind unter anderem als Farbfestiger oder Tönungsfestiger im Handel bekannt. Sie enthalten zusätzlich übliche für Haarfestiger bekannte, direkt auf das Haar aufziehende kosmetische Farbstoffe, wie beispielsweise aromatische Nitrofarbstoffe (zum Beispiel 1,4-Diamino-2-nitro-benzol, Pikraminsäure, 1-Hydroxy-2-amino-4-nitro-benzol und 1,4-Bis-[(2-hydroxyethyl)amino]-2-nitro-5-chlor-benzol), Azofarbstoffe (zum Beispiel C.I. 14 805-Acid Brown 4), Anthrachinonfarbstoffe (zum Beispiel C.I. 61 105-Disperse Violet 4) und Triphenylmethanfarbstoffe (zum Beispiel C.I. 42 535-Basic Violet 1), wobei die Farbstoffe dieser Klassen je nach der Art ihrer Substituenten sauren, nichtionogenen oder basischen Charakter haben können. Ihre Gesamtkonzentration in diesen Präparaten beträgt üblicherweise etwa 0,01 bis 2,0 Gewichtsprozent.

Die erfindungsgemäßen Mittel zur Festigung der Frisur weisen bei gleich guter Festigung des Haares gegenüber üblichen Mitteln eine besonders gute Kämmbarkeit und einen guten Griff des Haares im nassen Zustand sowie einen besonders angenehmen Griff des Haares im getrockneten Zustand auf.

Weiterhin können die erfindungsgemäßen Mittel Haarwaschmittel darstellen. Sie liegen dann in Form wäßriger Lösungen oder Emulsionen vor und enthalten neben dem N-Hydroxybutyl-chitosan zumindest ein anionisches, kationisches, nicht-ionisches oder amphoteres Tensid.

In diesen Haarwaschmitteln beträgt die Konzentration des Tensides im allgemeinen etwa 3 bis 50 Gewichtsprozent, vorzugsweise 3 bis 20 Gewichtsprozent, wobei der pH-Wert im allgemeinen zwischen 3 und 9, vorzugsweise zwischen 4 und 7, liegt.

Die erfindungsgemäßen Mittel, die in Form von Haarwaschmitteln vorliegen, enthalten im allgemeinen verschiedene Zusatzstoffe, insbesondere Parfüms, Konservierungsstoffe, Verdicker, Schaumstabilisatoren, Puffersubstanzen, kosmetische Harze, Pigmente und Farbstoffe.

Unter den Schaumstabilisatoren können die Fettamide und insbesondere die Mono- oder Diethanolamide von Kokosfettsäuren, Lauryl- oder Ölsäuremono- oder -diethanolamid, die zweckmäßigerwese in Mengen von 1 bis 10 und vorzugsweise von 1 bis 3 Gewichtsprozent Verwendung finden, angeführt werden.

Unter den Verdickern können insbesondere Acrylpolymere und Cellulosederivate, wie zum Beispiel Carboxymethylcellulose, Hydroxypropylmethylcellulose und Hydroxyethylcellulose, angeführt werden. Die Verdicker liegen im allgemeinen in einem Anteil von 0,1 bis 5 Gewichtprozent vor.

Unter den Tensiden oder oberflächenaktiven Agenzien, die in Kombination mit den neuen N-Hydroxybutyl-chitosanen verwendet werden, können beispielsweise die folgenden genannt werden:

a) die anionischen oberflächenaktiven Agenzien, wie beispielsweise die Alkali-, Erdalkali-, Ammonium- oder Alkanolaminsalze von Alkansulfonaten, Alkylsulfaten und Alkylethersulfaten, die $C_{12}$ bis $C_{18}$-Alkyl- und insbesondere $C_{12}$ bis $C_{14}$-Alkyl-Sulfatnatriumsalze oder -Triethanolaminsalze, die Natrium- oder Triethanolaminsalze von Lauryl- oder Tetradecylethersulfaten, das Dinatriumsalz des Sulfosuccinhalbesters von Alkanolamiden, die Seifen und die Polyethercarbonsäuren;

b) die nichtionischen oberflächenenaktiven Agenzien, wie beispielsweise oxethylierte Fettalkohole mit 12 bis 18 Kohlenstoffatomen, zum Beispiel mit bis zu 40 Mol Ethylenoxid pro Mol Fettalkohol oxethylierter Lauryl-, Tetradecyl-, Cetyl-, Oleyl- und Stearylalkohol, allein oder im Gemisch; die Fettalkohole von oxethyliertem Lanolin oder oxethyliertes Lanolin; Polyglycolether von gesättigten oder ungesättigten Fettalkoholen und Alkylphenolen mit 8 bis 30 Kohlenstoffatomen im Alkylrest und 1 bis 10 Glyceryleinheiten im Molekül; Fettsäurealkanolamide sowie oxethylierte Sorbitanfettsäureester;

c) die kationischen oberflächenaktiven Agenzien, wie beispielsweise das Dilauryldimethylammoniumchlorid, die Chloride oder Bromide von Alkyldimethylbenzylammonium, die Alkyltrimethylammoniumsalze, beispielsweise Cetyltrimethylammoniumchlorid oder -bromid, die Alkyldimethylhydroxyethylammoniumchloride oder -bromide, die Dialkyldimethylammoniumchloride oder -bromide, Alkylpyridiniumsalze, beispielsweise Lauryl- oder Cetylpyridiniumchlorid, die Alkylamidethyltrimethylammoniumethersulfate, Verbindungen mit kationischem Charakter wie Aminoxide, beispielsweise Alkyldimethylaminoxide oder Alkylaminoethyldimethylaminoxide;

d) die amphoteren oder zwitterionischen oberflächenaktiven Agenzien, wie beispielsweise die Carboxylderivate von Imidazol, die N-Alkylbetaine, die N-Alkylamidobetaine, die N-Alkylsulfobetaine, die N-Alkylaminoproponate, die Alkyldimethylammoniumacetate, die $C_{12}$ bis $C_{18}$ Alkyldimethylcarboxymethylammoniumsalze sowie die Fettsäurealkylamidobetaine, beispielsweise Dimethyl-carboxymethylenpropylenamido-stearat-betain.

Die erfindungsgemäßen kosmetischen Mittel können auch Cremes oder Lotionen zur Verwendung als Haarkur oder Hautpflegemittel darstellen. Sie liegen dann meist in Form von Öl-in-Wasser- oder Wasser-in-Öl-Emulsionen oder -Suspensionen vor und enthalten zusätzlich zu den neuen N-Hydroxybutylchitosanen kationische, nichtionogene, amphotere oder anionische Emulgatoren sowie als Bestandteil der Ölphase zum Beispiel Fettalkohole, Fettsäureester oder -amide, weiterhin Parfümöle, Vaseline, Wollfettalkohol oder feste beziehungsweise flüssige Paraffine.

Wenn die erfindungsgemäßen Mittel Haartönungs- oder Haarfärbemittel darstellen, so liegen sie ebenfalls bevorzugt in Form von Cremes oder Lotionen vor und enthalten zusätzlich übliche Haarfarbstoffe aus der Gruppe der aromatischen Nitrofarbstoffe, Azofarbstoffe, Anthrachinonfarbstoffe, Triphenylmethanfarbstoffe oder auch Oxidationsfarbstoffe, beispielsweise Resorcin und aromatische Diamine oder Aminophenole. Weiterhin können diese Mittel gegebenenfalls Alkalisierungsmittel, Antioxidantien sowie weitere für solche Mittel übliche kosmetische Zusätze und Hilfsstoffe enthalten.

Die erfindungsgemäßen Mittel können auch Dauerverformungsmittel oder Fixiermittel für Haare darstellen. Sie enthalten dann zusätzlich zu den genannten N-Hydroxybutyl-chitosanen Reduktionsmittel, wie zum Beispiel Thioglykolsäure, Thiomilchsäure und Ammoniumsulfit, oder Oxidationsmittel, wie zum Beispiel Hydrogenperoxid oder Natriumbromat, sowie gegebenenfalls Alkalisierungsagenzien oder Peroxidstabilisatoren, zum Beispiel Phosphorsäure, und andere kosmetische Hilfsstoffe und Zusatzstoffe, wie beispielsweise Parfümöle, Riechstoffe, Pflegestoffe und Farbstoffe.

Wie bereits erwähnt wurde, können die erfindungsgemäßen kosmetischen Mittel auch zur Behandlung der Haut verwendet werden.

In der Tat erleichtern diese kosmetischen Mittel die Befeuchtung der Haut, verhindern das Austrocknen und verleihen der Haut eine hervorragende Weichheit im Griff.

Die erfindungsgemäßen kosmetischen Mittel liegen hierzu vorzugsweise in Form von Cremes, Gelen, Emulsionen oder wäßrigen, alkoholischen oder wäßrig-alkoholischen Lösungen vor, die das N-Hydroxybutyl-chitosan in einer Konzentration von 0,1 bis 10 Gewichtsprozent und vorzugsweise von 0,2 bis 6 Gewichtsprozent enthalten.

Die im allgemeinen in diesen Kosmetikzubereitungen enthaltenen Hilfsstoffe sind beispielsweise Duftstoffe, Farbstoffe, Konservierungsmittel, Verdickungsmittel, Sequestriermttel, Emulgiermittel, Sonnenschutzfilter und ähnliche.

Diese Zubereitungen für die Hautbehandlung liegen insbesondere in Form von Cremes oder Lotionen zur Pflege der Hände oder des Gesichts oder in Fom von Sonnenschutzcremes, gefärbten Cremes, Abschminkmilchprodukten, Schaumbad- und Duschbad-Präparaten oder auch in Form von Desodorierzubereitungen vor.

Diese Zubereitungen werden unter Anwendung klassischer Verfahrensweisen hergestellt. Beispielsweise kann man zur Bildung einer Creme eine wäßrige Phase, die das erfindungsgemäße Chitosanderivat und gegebenenfalls andere Bestandteile oder Hilfstoffe gelöst enthält, und eine ölige Phase emulgieren. Für die ölige Phase kann man verschiedenartige Verbindungen verwenden, beispielsweise Paraffinöl, Vaselinöl, Süßmandelöl, Avocadoöl, Olivenöl, Fettsäureester, wie zum Beispiel Glycerylmonstearat, Ethylpalmitat und Isopropylpalmitat, oder Alkylmyristate, wie zum Beispiel Propylmyristat, Butylmyristat und Cetylmyristat. Man kann sie auch mit Fettsäurealkoholen, beispielsweise Stearyl- oder Cetylalkohol, oder Wachsen, beispielsweise Bienenwachs oder Wollwachs versetzen.

Die N-Hydroxybutyl-chitosanderivate können in diesen Kosmetikzubereitungen für die Hautpflege sowohl als Hauptwirkstoff als auch als Hilfsstoff enthalten sein.

Die in den erfindungsgemäßen kosmetischen Mitteln enthaltenen neuen Chitosanderivate leiten sich von Chitosan ab, einem Material, das durch Entacetylierung von Chitin, einem natürlich vorkommenden Acetylglucosamin, erhalten wird.

Das Chitosan ist im neutralen und alkalischen Medium unlöslich, es bildet jedoch auf Grund seiner chemischen Natur im sauren Medium mit organischen und anorganischen Säuren lösliche Salze. Diese finden beispielsweise in der Papier- und Textilindustrie als Additive Verwendung. Weiterhin werden sie als Koagulanzien für Suspensionen, als Chelatbildner für Schwermetallionen sowie in der Medizin und in der Kosmetik benutzt (siehe in diesem Zusammenhang die Veröffentlichung von Muzarelli: "Chitin", Pergamon Press, 1977).

Es sind bereits einige wasserlösliche Chitosanderivate bekannt, beispielsweise Carboxymethylchitosan (siehe Nud'ga, Plisko und Danilov, Zhur. Obsh. Khim. 43, No. 12, Seite 2752 bis 2756 (1973); SU-PS 325 234; sowie Okimasu, Nippon Nogei Kagaku Kaishi 32, Seite 383 bis 389 und 471 bis 473 (1958)) oder Sulfoethylchitosan (siehe Nud'ga, Plisko und Danilov, Zhur. Prikl. Khim. 47, No. 4, Seite 872 bis 875 (1974)). Diese wasserlöslichen Chitosanderivate sind jedoch entweder in ihrem ionischen Charakter verändert oder aber physiologisch bedenklich.

Hydroxyethylchitosan (Glykolchitosan) wurde von Senju und Okimasu (Nippon Nogei Kagaku Kaishi 23, Seite 423 bis 437 (1950)) bei der Glykolierung von Chitin in Gegenwart starken Alkalis durch gleichzeitige Entacetylierung erhalten.

Aufgrund niedriger Substitutionsgrade beziehungsweise Quervernetzung wasserunlösliche Hydroxyalkylderivate des Chitosans, deren stark wasserabsorbierende Eigenschaften anwendungstechnisch von Interesse sind, werden in der JP-PS 54-11 955 von 1979 erwähnt.

Schließlich beschreibt die JP-PS 57-180 602 (1982) die Synthese wasserlöslicher Chitosanderivate, die durch Reaktion von Alkylenoxiden mit Chitosan in Anwesenheit von Alkali in einem Gemisch von Wasser und einem organischen Lösungsmittel erhalten werden.

All diesen mehr oder weniger wasserlöslichen beziehungsweise wasserquellbaren Derivaten liegt die Umsetzung des Chitosans mit Alkylierungsmitteln in Gegenwart starken Alkalis zugrunde, die unter den gewählten Reaktionsbedingungen ausschließlich oder überwiegend eine O-Substitution zur Folge hat. Die zur 0-Alkylierung notwendige Anwesenheit von Alkali bestimmt jedoch nicht nur den Substitutionsort, sondern bewirkt darüber hinaus, insbesondere bei höheren Temperaturen, einen Abbau der Polymerkette. Desweiteren stellen die nach der Reaktion durch Neutralisation des überschüssigen Alkalis entstehenden Salze Nebenprodukte dar, die weitere Reinigungsschritte notwendig machen.

Im Gegensatz hierzu berichtet die DE-OS 3 223 423 sowie die EP-OS 0 097 229 von wasserlöslichen N-substituierten Chitosanderivaten, die in bevorzugter Weise durch die Reaktion einer wäßrigen Dispersion von Chitosan mit Glycid erhalten werden. Die rasche Hydrolyse des Glycids in Gegenwart von Wasser, sein hoher Preis und die Tatsache, daß Glycid nicht großtechnisch hergestellt wird, verteuern jedoch das Verfahren zur Herstellung dieser Derivate.

Überträgt man die in der DE-OS 3 223 423 angegebenen, bevorzugt gewählten Reaktionsbedingungen auf die Umsetzung von Chitosan mit Butylenoxid, so erhält man keine wasserlöslichen Derivate.

Überraschenderweise wurde nunmehr gefunden, daß sich bei Verwendung von Gemischen aus Wasser und organischen Lösungsmitteln Chitosan auf einfache Weise mit Butylenoxid zu Hydroxybutylderivaten mit besonders vorteilhaften Film- und Löslichkeitseigenschaften umwandeln läßt.

In Abwesenheit basischer Katalysatoren kommt es hierbei zur Substitution der freien Aminogruppen, was durch Ermittlung des primären Amin-Stickstoff nach van Slyke (siehe K.H. Bauer und H. Moll, "Die organische Analyse", 2. Aufl., Seite 170 bis 172, Akademische Verlagsgesellschaft Geest und Portig KG, Leipzig 1950, und H. Roth, E. v. Hulle et al. in "Analytische Methoden", Seite 674 bis 676, Georg Thieme Verlag, Stuttgart 1953) sowie durch $^{13}C$–NMR bestätigt wird.

In dem Derwent Japanese Patent Report, Nr. 76-17 481 X, 1976 (SEKISUI CHEMICAL K.K.) werden unter anderem auch Hydroxyalkyl-chitosane (beispielsweise Hydroxybutylchitosane) genannt, es ist jedoch aus dieser Schrift nicht ersichtlich, ob es sich hierbei um N-Hydroxyalkylchitosane oder O-Hydroxyalkylchitosane handelt.

Da die dort genannten Hydroxyalkyl-chitosane jedoch für die Herstellung von semipermeablen Membranen verwendet werden sollen und derartige Membranen unlöslich sein müssen, müssen diese unlöslichen Hydroxyalkyl-chitosane eine andere Struktur besitzen, als die der vorliegenden Erfindung zugrunde liegenden, in Wasser und organischen Lösungsmitteln löslichen, N-Hydroxybutyl-chitosane.

Gegenstand der vorliegenden Erfindung sind daher ferner sowohl wasser- als auch alkohollösliche, vom Chitosan abgeleitete N-Hydroxybutyl-chitosane und deren lösliche Salze mit organischen oder anorganischen Säuren, bestehend aus

a) 4 bis 40 Molprozent Monomereinheiten der Formel (I)

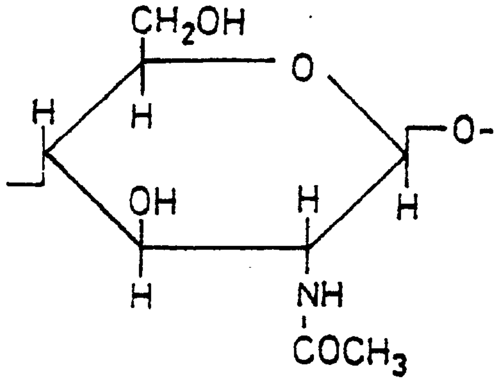

(I)

und

b) 60 bis 96 Molprozent Monomereinheiten der Formel (II)

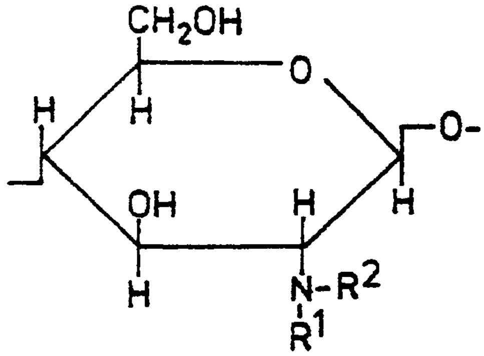

(II),

wobei $R^1$ und $R^2$ gleich oder verschieden sein können und entweder Wasserstoff oder die Gruppe

$$\left[-CH_2-\underset{\mid \atop O-}{CH}-CH_2-CH_3\right]_n H,$$

mit n gleich eine ganze Zahl von 1 bis 5 bedeuten, mit der Bedingung, daß bei mindestens 50 Prozent der Einheiten der Formel (II) $R^1$ und $R^2$ nicht gleichzeitig Wasserstoff darstellen.

Die neuen Chitosan-Derivate werden erfindungsgemäß erhalten, indem man eine Aufschlämmung von Chitosan (zu 60 bis 96 Prozent entacetyliertes Chitin) oder seiner Salze bei Temperaturen zwischen 20 und 120 Grad Celsius, vorzugsweise zwischen 40 und 100 Grad Celsius, unter Druck im Autoklaven, in einem geeigneten Verhältnis mit Butylenoxid (1,2-Epoxybutan) über einen Zeitraum von 3 bis 72 Stunden, vorzugsweise 6 bis 48 Stunden, umsetzt.

In bevorzugter Weise führt man die Reaktion in einem Gemisch bestehend aus Wasser und einem organischen Lösungsmittel in neutralem Medium aus. Bei Verwendung von Chitosansalzen oder von Chitosan in Anwesenheit saurer Katalysatoren, wie zum Beispiel Salzsäure, kann die Reaktion in einer Dispersion oder Lösung bestehend aus Wasser und einem organischen Lösungsmittel oder Wasser und überschüssigem Butylenoxid erfolgen. Das molare Verhältnis Chitosan zu Butylenoxid wählt man zwischen 1 : 3 bis 1 : 15.

Nach beendeter Reaktion entfernt man das überschüssige Alkylierungsmittel, trennt eventuell vorhandene unlösliche Anteile aus den Lösungen des Chitosanderivates durch Filtration ab, neutralisiert gegebenenfalls, engt am Rotationsverdampfer ein und fällt die Chitosanderivate unmittelbar oder nach der Dialyse in Aceton aus.

Die Salze der erfindungsgemäßen N-Hydroxybutyl-chitosane können durch Neutralisierung der Aminogruppen der N-Hydroxybutyl-chitosane mit anorganischen oder organischen Säuren erhalten werden. Gemäß der vorliegenden Erfindung sind jedoch nur solche Salze verwendbar, die in Wasser löslich sind Geeignete Salze sind zum Beispiel solche, die mit Salzsäure, Glykolsäure, Milchsäure, Ameisensäure, Zitronensäure oder Essigsäure gebildet werden.

Die nachfolgenden Beispiele sollen den Gegenstand der Erfindung näher erläutern, ohne diesen hierauf zu beschränken.

**Herstellungsbeispiele**

**Beispiel 1**

50 g (0,31 mol) niedermolekulares, gemahlenes Chitosan mit einer Grenzviskositätszahl (Eta) von 160 ml/g und einem Entacetylierungsgrad von 90 Prozent werden in 200 ml Isopropanol / Wasser (1 : 1) dispergiert und im Autoklaven mit 64,9 g (0,9 mol) Butylenoxid versetzt. Nach 12-stündiger Reaktionszeit bei 100 Grad Celsius entfernt man das überschüssige Alkylierungsmittel durch Hindurchleiten von Stickstoff oder durch kurzzeitiges Erwärmen zum Sieden unter dem Abzug, nachdem man das hochviskose Umsetzungsprodukt mit Isopropanol im Verhältnis 1 : 1 verdünnt hat.

Nach Druckfiltration zur Entfernung nicht umgesetzter Anteile engt man das Filtrat am Rotationsverdampfer ein und fällt schließlich in der 8 bis 10-fachen Menge Aceton aus.

Das ausgefällte Derivat wird mittels eines Ultra-Turax fein dispergiert, um eingeschlossene Butylenglykole zu entfernen.

Der Niederschlag wird auf einer Glassinternutsche gesammelt, gründlich mit Aceton gewaschen und bei 50 Grad Celsius im Vakuumtrockenschrank getrocknet.

Es werden 45 g N-Hydroxybutylchitosan erhalten. Kenndaten:
Grenzviskositätszahl (Eta)= 62 ml/g
Substitutionsgrad Hydroxybutyl= 2,1
Pendelhärte= 207 sec.
Wasserdampfaufnahme= 5,8 Prozent

**Beispiel 2**

20 g (0,12 mol) eines hochmolekularen Chitosans, mit einer Grenzviskositätszahl (Eta) von 1600 ml/g und einem Entacetylierungsgrad von 76 Prozent werden in einem Druckgefäß in einem Gemisch von 800 ml Ethanol/Wasser (1 : 1) dispergiert, mit 77,9 g (1,08 mol) Butylenoxid versetzt und unter Rühren bei 80 Grad Celsius über 20 Stunden zur Reaktion gebracht. Die Aufarbeitung erfolgt wie in Beispiel 1 beschrieben.

Die Ausbeute an N-Hydroxybutyl-chitosan beträgt 25,4 g. Grenzviskositätszahl (Eta)= 215 ml/g
Substitutionsgrad Hydroxybutyl= 1,5
Pendelhärte= 176 sec
Wasserdampfaufnahme= 7,1 Prozent

**Beispiel 3**

50 g (0,3 mol) niedermolekulares, gemahlenes Chitosan mit einer Grenzviskositätszahl (Eta) von 160 ml/g und einem Entacetylierungsgrad von 90 Prozent werden unter Zugabe von 43,8 g (0,3 mol) 25-prozentiger Salzsäure in einem Gemisch von je 100 ml Isopropanol und Wasser dispergiert und in einem Autoklaven mit 129,8 g (1,8 mol) Butylenoxid 24 Stunden lang bei 90 Grad Celsius umgesetzt.

Die Aufarbeitung des Umsetzungsproduktes erfolgt nach der Neutralisation und Dialyse wie in Beispiel 1 beschrieben.

Es werden 55 g N-Hydroxybutyl-chitosan erhalten.

Kenndaten:
Grenzviskositätszahl (Eta)= 63 ml/g
Substitutionsgrad Hydroxybutyl= 2,0
Pendelhärte= 200 sec
Wasserdampfaufnahme= 4,1 Prozent

Der Substitutionsgrad für die Hydroxybutylreste wurde mit Hilfe der $^1$H-NMR-Spektren ermittelt.

Die Messung der Grenzviskositäten erfolgte in einer wäßrigen Lösung von 0,2 mol/l Essigsäure und 0,1 mol/l Natriumacetat (Chitosan) beziehungsweise in einer wäßrigen Lösung von 0,2 mol/l Essigsäure und 0,1 mol/l Natriumchlorid (N-Hydroxybutylchitosan) bei 25 Grad Celsius unter Verwendung eines DIN-Ubbelohde-Viskosimeters.

Die Pendelhärte wurde nach König (W. König, "Härtemessungen mit dem Pendelhärteprüfer", Farbe und Lack 65, Seite 435 bis 443 (1959); DIN 53 157) bestimmt.

Die Wasseraufnahme wurde bei 70 Prozent relativer Luftfeuchte gegenüber 30 Prozent relativer Luftfeuchte ermittelt.

**Beispiele für kosmetische Mittel**

**Beispiel 4** Haarspray

| | |
|---|---|
| 3,5 g | N-Hydroxybutyl-chitosan nach Beispiel 3 (Eta = 63 ml/g, Substitutionsgrad = 2,0) |
| 0,6 g | Parfümöl |
| 95,9 g | Ethanol (96-prozentig) |
| 100,0 g | |

Abfüllverhältnis:
26,8 Prozent Wirkstoff
53,2 Prozent FCCl3
20,0 Prozent Propan/Butan (Druck: 0,27 MPa bei 20 Grad Celsius)

**Beispiel 5** Haarfestiger

| | |
|---|---|
| 0,6 g | N-Hydroxybutyl-chitosan nach Beispiel 1 (Eta = 62 ml/g, Substitutionsgrad = 2,1) |
| 25,0 g | Isopropanol |
| 0,4 g | Ameisensäure (10-prozentige, wäßrige Lösung) |
| 0,2 g | Parfümöl |
| 73,8 g | Wasser |
| 100,0 g | |

20 ml dieser Lösung werden auf gewaschenes, handtuchtrockenes Haar verteilt. Anschließend werden die Haare in üblicher Weise zur Frisur eingelegt und getrocknet. Bei guter Festigungswirkung zeigt das Haar, im Vergleich zu einem Haarfestiger auf der Basis von Chitosan/Ameisensäure, einen angenehmeren und weicheren Griff.

**Beispiel 6** Sprühfönlotion

| | |
|---|---|
| 0,5 g | N-Hydroxybutyl-chitosan nach Beispiel 3 (Eta = 63 ml/g, Substitutionsgrad = 2,0) |
| 58,0 g | Isopropanol |
| 0,1 g | Cetyltrimethylammoniumchlorid |
| 0,4 g | Phthalsäurediethylester |
| 0,4 g | Parfümöl |
| 40,6 g | Wasser |
| 100,0 g | |

Abfüllverhältnis:
91 Prozent Wirkstoff
9 Prozent Propan/Butan (Druck: 0,27 MPa bei 20 Grad Celsius)

Die Fönlotion wird auf das gewaschene, handtuchtrockene Haar aufgesprüht. Anschließend wird das Haar in üblicher Weise gefönt und geformt. Das Haar zeigt bei guter Konditionierung gegenüber einer Fönlotion mit synthetischen Polymeren einen wesentlich angenehmeren und weicheren Griff.

**Beispiel 7** Tönungsfestiger

| | | |
|---|---|---|
| 0,60 | g | N-Hydroxybutyl-chitosan nach Beispiel 1 (Eta = 62 ml/g, Substitutionsgrad = 2,1) |
| 0,15 | g | 1,4-Bis-[(2-hydroxyethyl)-amino]-2-nitro-5-chlor-benzol |
| 45,00 | g | Ethanol |
| 54,25 | g | Wasser |
| 100,00 | g | |

20 ml dieser Lösung werden auf die gewaschenen, handtuchtrockenen Haare gegeben, sodann wird das Haar eingelegt und getrocknet. Die Haare sind rot-violett gefärbt und gefestigt.

**Beispiel 8** Anionisches Haarwaschmittel

| | | |
|---|---|---|
| 1,00 | g | N-Hydroxybutyl-chitosan nach Beispiel 2 (Eta = 215 ml/g, Substitutionsgrad = 1,5) |
| 40,00 | g | Laurylalkoholdiglykolethersulfat-Natriumsalz (28-prozentige, wäßrige Lösung) |
| 4,00 | g | Natriumchlorid |
| 0,05 | g | Farbstoff |
| 0,10 | g | Formaldehyd (25-prozentige, wäßrige Lösung) |
| 54,85 | g | Wasser |
| 100,00 | g | |

Es wird ein klares Shampoo erhalten. Das damit gewaschene Haar ist hinsichtlich Griff, Glanz und Kämmbarkeit ausgezeichnet konditioniert.

**Beispiel 9** Amphoteres, tönendes Haarwaschmittel

| | | |
|---|---|---|
| 2,00 | g | N-Hydroxybutyl-chitosan nach Beispiel 1 (Eta = 62 ml/g, Substitutionsgrad = 2,1) |
| 40,00 | g | Dimethyl-carboxymethylen-propylenamido-stearat-betain (35-prozentige, wäßrige Lösung) |
| 5,06 | g | Ameisensäure (10-prozentige, wäßrige Lösung) |
| 1,00 | g | Pikraminsäure (einprozentige, wäßrige Lösung) |
| 3,50 | g | Kokosfettsäure (einprozentige, wäßrige Lösung) |
| 48,44 | g | Wasser, vollentsalzt |
| 100,00 | g | |

Mit 15 bis 20 g des obigen Haarwaschmittels wird das Haar einshampooniert. Nach einer Einwirkungszeit von 5 bis 10 Minuten spült man mit Wasser aus. Das Haar ist gelb-orange getönt und ausgezeichnet konditioniert.

**Beispiel 10** Kationisches Haarkurmittel

| | | |
|---|---|---|
| 0,30 | g | N-Hydroxybutyl-chitosan nach Beispiel 2 (Eta = 215 ml/g, Substitutionsgrad = 1,5) |
| 4,00 | g | Cetylstearylalkohol |
| 1,48 | g | Milchsäure (10-prozentige, wäßrige Lösung) |
| 2,50 | g | Kokos(pentaethoxy)methylammoniumchlorid |
| 1,00 | g | Sorbitanmonopalmitat, mit 20 Mol Ethylenoxid oxethyliert |
| 90,72 | g | Wasser, vollentsalzt |
| 100,00 | g | |

35 g des Haarkurmittels nach Beispiel 10 werden im gewaschenen Haar verteilt und nach einer Einwirkungszeit von 3 bis 5 Minuten mit Wasser wieder ausgespült. Als Ergebnis werden ausgezeichneter Griff, Glanz sowie Kämmbarkeit des Haares erhalten.

**Beispiel 11** Haarkurmittel, gelförmig

| | | |
|---|---|---|
| 2,1 | g | N-Hydroxybutyl-chitosan nach Beispiel 2 (Eta = 215 ml/g, Substitutionsgrad = 1,5) |
| 0,6 | g | Hydroxypropylmethylcellulose |
| 0,5 | g | Laurylpyridiniumchlorid |
| 96,8 | g | Wasser, vollentsalzt |
| 100,0 | g | (auf pH 5,0 mit zehnprozentiger Ameisensäure eingestellt) |

Die Anwendung des Gels erfolgt wie in Beispiel 9 beschrieben wurde. Als Ergebnis werden Griff, Glanz und Kämmbarkeit des Haares wesentlich verbessert.

**Beispiel 12** Haartönungsmittel

| | | |
|---|---|---|
| 0,30 | g | N-Hydroxybutyl-chitosan nach Beispiel 1 (Eta = 62 ml/g, Substitutionsgrad = 2,1) |
| 12,00 | g | Cetylstearylalkohol |
| 0,10 | g | 4-Hydroxy-benzoesäureethylester |
| 6,00 | g | Laurylalkoholdiglykolethersulfat-Natriumsalz (28-prozentige, wäßrige Lösung) |
| 0,50 | g | Parfümöl |
| 0,50 | g | 1-Hydroxy-2-amino-4-nitro-benzol |
| 0,85 | g | 1,4-Diamino-2-nitro-benzol |
| 0,24 | g | Natriumhydroxid |
| 79,51 | g | Wasser |
| 100,00 | g | |

30 bis 40 g des obigen Haartönungsmittels werden in dem gewaschenen Haar verteilt und nach einer Einwirkungszeit von etwa 20 Minuten ausgespült. Das Haar ist rötlich gefärbt und weist eine gute Kämmbarkeit und einen angenehmen Griff auf.

**Beispiel 13** Oxidationshaarfärbemittel

| | | |
|---|---|---|
| 0,50 | g | N-Hydroxbutyl-chitosan nach Beispiel 2 (Eta = 215 ml/g, Substitutionsgrad = 1,5) |
| 0,08 | g | 3,5-Diamino-2,6-dimethoxy-pyridin-dihydrochlorid |
| 0,30 | g | 1,4-Diamino-benzol |
| 0,25 | g | Resorcin |
| 0,30 | g | Natriumsulfit |
| 3,50 | g | Laurylalkoholdiglykolethersulfat-Natriumsalz (28-prozentige, wäßrige Lösung) |
| 15,00 | g | Cetylalkohol |
| 3,00 | g | Ammoniak |
| 77,07 | g | Wasser |
| 100,00 | g | |

50 g dieses Haarfärbemittels werden mit 50 ml 6-prozentiger Hydrogenperoxidlösung gemischt und auf weißes Haar aufgetragen. Nach 30 Minuten wird das Haar mit Wasser ausgespült und getrocknet. Das Haar hat eine natürlich wirkende matt-blonde Färbung sowie einen natürlichen, angenehmen Griff erhalten.

**Beispiel 14** Dauerwellmittel

| | |
|---|---|
| 0,5 g | N-Hydroxybutyl-chitosan nach Beispiel 3 (Eta = 63 ml/g, Substitutionsgrad = 2,0) |
| 10,0 g | Thioglykolsäure |
| 8,0 g | Ammoniak (25-prozentige, wäßrige Lösung) |
| 6,1 g | Ammoniumhydrogencarbonat |
| 75,4 g | Wasser |
| 100,0 g | |

Zur Anwendung trägt man dieses Dauerwellmittel auf das gewickelte, handtuchtrockene Haar gleichmäßig auf und läßt es etwa 20 Minuten lang einwirken. Danach wird das Haar mit Wasser ausgespült und in bekannter Weise oxidativ behandelt. Es wird ein gutes Wellergebnis erhalten, und die Haare fühlen sich natürlich und weich an.

**Beispiel 15** Hautcreme

| | |
|---|---|
| 0,30 g | N-Hydroxybutyl-chitosan nach Beispiel 1 (Eta = 62 ml/g, Substitutionsgrad = 2,1) |
| 3,00 g | Stearylalkohol |
| 1,00 g | Wollwachs (Adeps Lanae) |
| 1,00 g | Vaseline |
| 0,76 g | Milchsäure (10-prozentige, wäßrige Lösung) |
| 1,00 g | Natriumacetylstearylsulfat |
| 92,94 g | Wasser, vollentsalzt |
| 100,00 g | |

Alle Prozentangaben in der vorliegenden Anmeldung stellen Gewichtsprozente dar.

## Patentansprüche

1. Kosmetisches Mittel zur Behandlung von Haaren oder der Haut, dadurch gekennzeichnet, daß es in einer geeigneten Kosmetikgrundlage ein N-Hydroxybutylchitosan, bestehend aus

a) 4 bis 40 Molprozent Monomereinheiten der Formel (I)

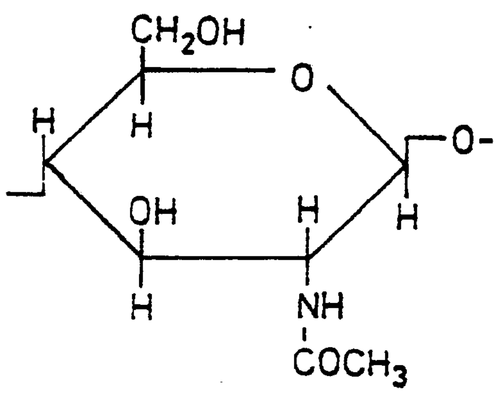

(I)

und

b) 60 bis 96 Molprozent Monomereinheiten der Formel (II)

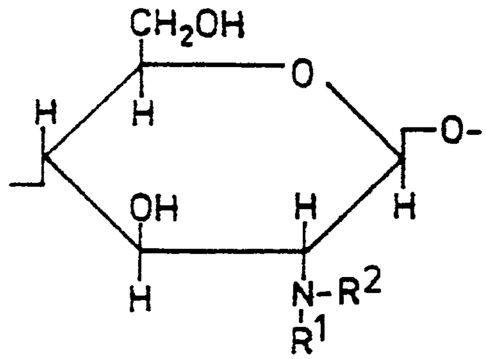

(II),

worin $R^1$ und $R^2$ gleich oder verschieden sind und Wasserstoff oder die Gruppe

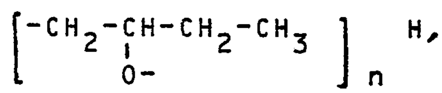

mit n gleich eine ganze Zahl von 1 bis 5, bedeuten, unter der Bedingung, daß bei mindestens 50 Prozent der Einheiten der Formel (II) $R^1$ und $R^2$ nicht gleichzeitig Wasserstoff darstellen, oder dessen lösliches Salz enthält.

2. Mittel nach Anspruch 1, dadurch gekennzeichnet, daß es das N-Hydroxybutyl-chitosan in einer Menge von 0,05 bis 10,0 Gewichtsprozent enthält.

3. Mittel nach Anspruch 1 und 2, dadurch gekennzeichnet, daß es in Form einer wäßrigen, alkoholischen oder wäßrig-alkoholischen Zubereitung, insbesondere als Lösung, Creme, Gel, Dispersion oder Emulsion, vorliegt.

4. Mittel nach Anspruch 1 bis 3, dadurch gekennzeichnet, daß der pH-Wert 2 bis 11 beträgt.

5. Mittel nach Anspruch 1 bis 4, dadurch gekennzeichnet, daß es zusätzlich ein bekanntes filmbildendes synthetisches oder natürliches kosmetisches Polymer enthält.

6. Mittel nach Anspruch 1 bis 5, dadurch gekennzeichnet, daß es zusätzlich mindestens einen kosmetischen Farbstoff in einer Konzentration von 0,01 bis 2,0 Gewichtsprozent enthält und in Form eines Farbfestigers oder Tönungsfestigers vorliegt.

7. Mittel nach Anspruch 1 bis 6, dadurch gekennzeichne daß es als Kosmetikgrundlage eine wäßrige, alkoholische oder wäßrig-alkoholische Zubereitung enthält, die mit einem unter Druck verflüssigten Treibmittel vermischt ist, in einem Druckbehälter abgefüllt ist und in Form eines Aerosolsprays oder -schaums vorliegt.

8. Mittel nach Anspruch 1 bis 4, dadurch gekennzeichnet, daß es zusätzlich mindestens ein kationisches, nichtionisches, amphoteres oder anionisches Tensid enthält und in Form eines Haarwaschmittels vorliegt.

9. Mittel nach Anspruch 8, dadurch gekennzeichnet, daß es das Tensid in einer Konzentration von 3 bis 50 Gewichtsprozent enthält und einen pH-Wert zwischen 3 und 9 aufweist.

10. Makromolekulare, vom Chitosan abgeleitete N-Hydroxybutylverbindung bestehend aus

a) 4 bis 40 Molprozent Monomereinheiten der Formel (I)

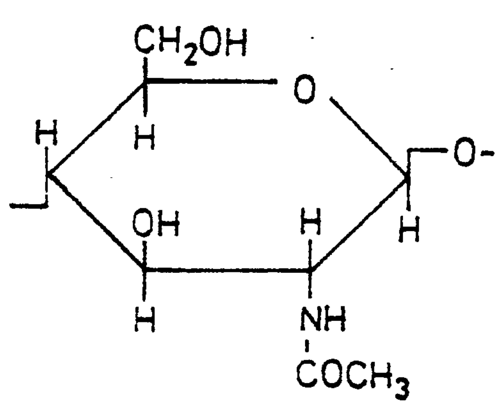

(I)

und

b) 60 bis 96 Molprozent Monomereinheiten der Formel (II)

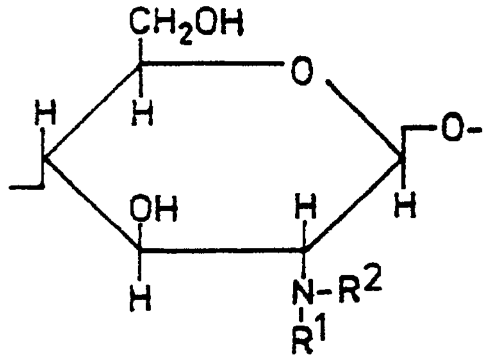

(II),

wobei $R^1$ und $R^2$ gleich oder verschieden sind und entweder Wasserstoff oder die Gruppe

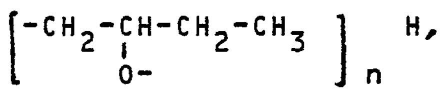

mit n gleich einer ganzen Zahl von 1 bis 5, bedeuten, mit der Bedingung, daß bei mindestens 50 Prozent der Einheiten der Formel (II) $R^1$ und $R^2$ nicht gleichzeitig Wasserstoff darstellen, oder deren lösliches Salz.

11. Verfahren zur Herstellung der Verbindungen nach Anspruch 10, dadurch gekennzeichnet, daß man eine Aufschlämmung eines Chitosan, bestehend aus 60 bis 96 Prozent entacetyliertem Chitin, oder dessen Salz bei 20–120°C 3 bis 72 Stunden lang mit Butylenoxid im geeigneten Verhältnis zur Umsetzung bringt.

12. Verfahren nach Anspruch 11, dadurch gekennzeichnet, daß man Chitosan mit Butylenoxid in einem Gemisch bestehend aus Wasser und einem organischen Lösungsmittel in neutralem Medium zur Reaktion bringt.

13. Verfahren nach Anspruch 11, dadurch gekennzeichnet, daß man das Chitosansalz oder Chitosan in Gegenwart saurer Katalysatoren in einer Dispersion oder Lösung bestehend aus Wasser und einem organischen Lösungsmittel oder Wasser und überschüssigem Butylenoxid zur Reaktion bringt.

14. Verfahren nach Anspruch 11 bis 13, dadurch gekennzeichnet, daß, bezogen auf die eingesetzte Menge Chitosan, das Butylenoxid in einem drei- bis fünfzehnfachen Überschuß eingesetzt wird.

**Claims**

1. Cosmetic composition for treating hair or skin, characterised in that it contains in a suitable cosmetic base, a N-hydroxy butyl chitosan, consisting of

a) 4 to 40% mol monomer units of the formula (1),

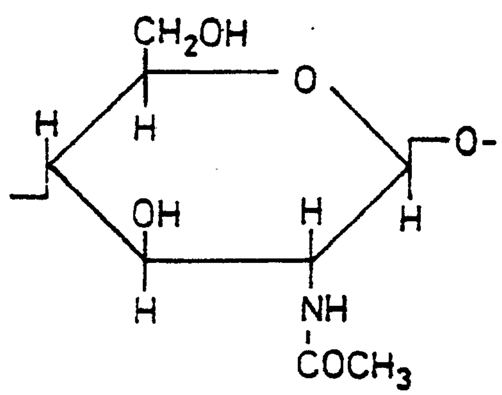

(I)

and

b) 60 to 96% mol monomer units of formula (II)

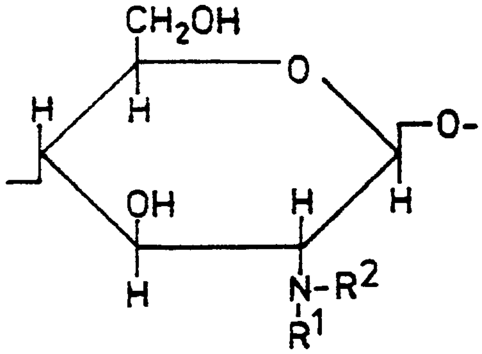

(II),

in which $R^1$ and $R^2$ are equal or different and represent either hydrogen or the group

$$\left[-CH_2-\underset{\displaystyle O-}{CH}-CH_2-CH_3\right]_n H,$$

with n being equal to an integer from 1 to 5 on condition that in the case of at least 50% of the units of formula (II) $R^1$ and $R^2$ do not represent hydrogen at the same time, or its soluble salt.

2. Composition according to claim 1, characterised in that N-hydroxy butyl chitosan is contained in a quantity of 0.05 to 10.0% by weight.

3. Composition according to claims 1 and 2, characterised in that it is in the form of an aqueous, alcoholic or aqueous-alcoholic preparation, in particular as a solution, cream, gel, dispersion or emulsion.

4. Composition according to claims 1 to 3, characterised in that the pH value is 2 to 11.

5. Composition according to claims 1 to 4, characterised in that it contains in addition a know film-forming synthetic or natural cosmetic polymer.

6. Composition according to claims 1 to 5, characterised in that it contains in addition at least a cosmetic colouring matter in a concentration of 0.01 to 2.0% by weight and is present in the form of a colour strengthener or tinting strengthener.

7. Composition according to claims 1 to 6, characterised in that it contains an aqueous, alcoholic or aqueous-alcoholic preparation as a cosmetic base, which is mixed with a propellant liquified under pressure, filled into a pressure container and is present in the form of an aerosol spray or foam.

8. Composition according to claims 1 to 4, characterised in that it contains in addition at least one cationic, non-ionic, amphoteric or anionic tenside and is present in the form of a shampoo.

9. Composition according to claim 8, characterised in that it contains the tenside in a concentration of 3 to 50% by weight and has a pH value of between 3 and 9.

10. Macromolecular N-hydroxybutyl compound derived from chitosan consisting of

a) 4 to 40% mol monomer units of formula (I)

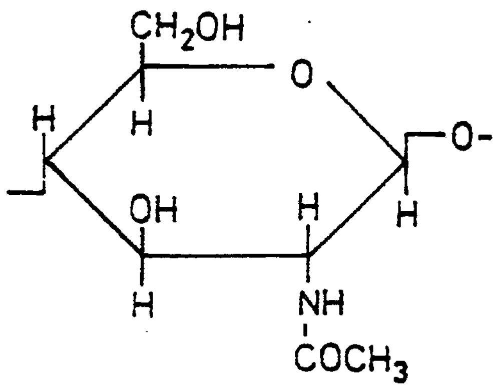

(I)

and

b) 60 to 96% mol monomer units of formula (II)

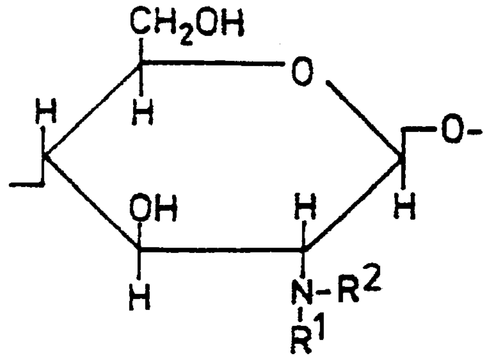

(II),

in which $R^1$ and $R^2$ are equal or different and represent either hydrogen or the group

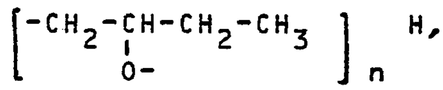

with n being equal to an integer from 1 to 5 on condition that in the case of at least 50% of the units of formula (II) $R^1$ and $R^2$ do not represent hydrogen at the same time, or its soluble salt.

11. Process for manufacturing the compounds according to claim 10, characterised in that a slurry of chitosan, consisting of 60 to 96% deacetylated chitin, or its salt, is made to react with butylene oxide in a suitable ratio for 3 to 72 hours at a temperature of 20 to 120°C.

12. Process according to claim 11, characterised in that chitosan with butylene oxide is made to react in a mixture consisting of water and a organic solvent in a neutral medium.

13. Process according to claim 11, characterised in that the chitosan salt or chitosan is made to react in the presence of acid catalysts in a dispersion or solution consisting of water and an organic solvent or water excess butylene oxide.

14. Process according to claims 11 to 13, characterised in that the butylene oxide is introduced as a three-to fifteenfold excess in relation to the quantity of chitosan used.

**Revendications**

1. Procédé cosmétique pour le traitement des cheveux ou de la peau, caractérisé en ce qu'il renferme, dans un substrat de cosmétique approprié, un N-hydroxybutyl-chitosane, comprenant

a) un pourcentage molaire de 4 à 40% d'unités monomères répondant à la formule (I)

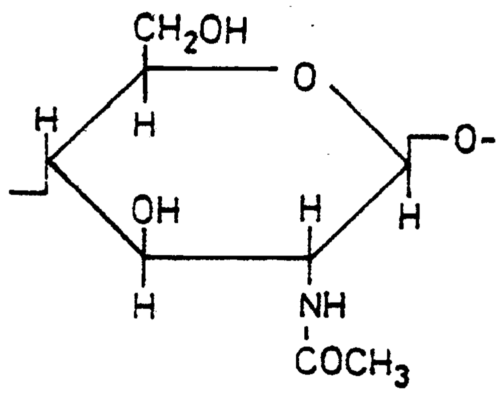

(I)

et

b) un pourcentage molaire de 60 à 96% d'unités monomères répondant à la formule (II)

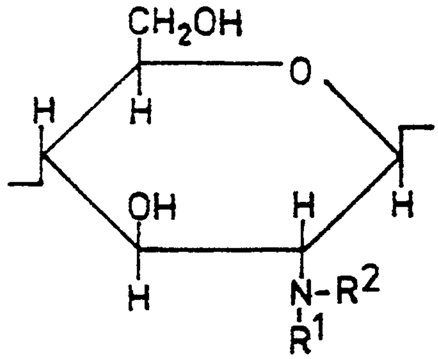

(II),

dans laquelle $R^1$ et $R^2$ sont identiques ou différents et représentent l'hydrogène ou le groupe

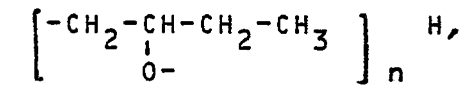

n étant égal à un nombre entier de 1 à 5, sous la condition que pour au moins 50% des unités répondant à la formule (II), $R^1$ et $R^2$ ne représentent pas en même temps l'hydrogène, ou un sel soluble dans l'eau de celui-ci.

2. Procédé selon la revendication 1, caractérisé en ce qu'il renferme le N-hydroxybutyl-chitosane dans une proportion en poids de 0,05 à 10,0%.

3. Procédé selon les revendications 1 et 2, caractérisé en ce qu'il se présente sous la forme d'une préparation aqueuse, alcoolique ou dans de l'eau et de l'alcool, en particulier d'une solution, d'une crème, d'un gel, d'une dispersion ou d'une émulsion.

4. Procédé selon les revendications 1 à 3, caractérisé en ce que la valeur de pH est de 2 à 11.

5. Procédé selon les revendications 1 et 4, caractérisé en ce qu'il renferme, en plus, un polymère cosmétique filmogène connue, synthétique ou naturel.

6. Procédé selon les revendications 1 et 5, caractérisé en ce qu'il renferme, en plus, au moins un colorant cosmétique à une concentration de 0,01 à 2,0% en poids et se présente sous la forme d'un fixateur de couleur ou d'un fixateur de nuance.

7. Procédé selon les revendications 1 à 6, caractérisé en ce qu'il renferme, comme substrat cosmétique, une préparation aqueuse, alcoolique ou dans de l'eau et de l'alcool, qui est mélangée avec un agent moussant liquéfié sous pression, est transvasé dans un récipient sous pression et se présente sous la forme d'un spray ou d'une mousse d'aérosol.

8. Produit selon les revendications 1 à 4, caractérisé en ce qu'il renferme, en plus, au moins un agent tension-actif cationique, non ionique, amphotère ou anionique et se présente sous la forme d'un produit de lavage des cheveux.

9. Produit selon la revendication 8, caractérisé en ce qu'il renferme l'agent tensio-actif à une concentration de 3 à 50% en poids et présente une valeur de pH comprise entre 3 et 9.

10. Composé N-hydroxybutylique macromoléculaire dérivant du chitosane, comprenant

a) un pourcentage molaire de 4 à 40% d'unités monomères répondant à la formule (I)

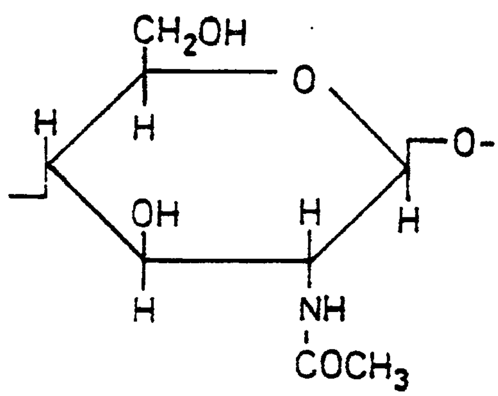

(I)

et

b) un pourcentage molaire de 60 à 96% d'unités monomères répondant à la formule (II)

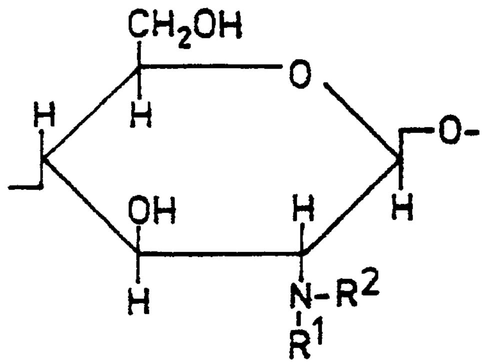

(II),

$R^1$ et $R^2$ étant identiques ou différents et représentent l'hydrogène ou le groupe

$$\left[-CH_2-\underset{\substack{| \\ O-}}{CH}-CH_2-CH_3\right]_n H,$$

n étant égal à un nombre entier de 1 à 5, sous la condition que, pour au moins 50% des unités répondant à la formule (II), $R^1$ et $R^2$ ne représentent pas en même temps l'hydrogène, ou un sel soluble de ce composé.

11. Procédé de préparation des composés selon la revendication 10, caractérisé en ce qu'on fait réagir une suspension du chitosane, coomprenant 60 à 96% de chitine désacétylée, ou un sel de celui-ci, sur de l'oxyde de butylène, dans une proportion appropriée 3 à 72 heures à une température de 20 à 120 dégrés Celsius.

12. Procédé selon la revendication 11, caractérisé en ce qu'on fait réagir du chitosane sur de l'oxyde de butylène dans un mélange comprenant de l'eau et une solvant organique en milieu neutre.

13. Procédé selon la revendication 11, caractérisé en ce qu'on fait réagir le sel de chitosane ou le chitosane en présence de catalyseurs acides dans une dispersion ou une solution comprenant de l'eau et un solvant organique ou de l'eau et de l'oxyde de butylène en excès.

14. Procédé selon les revendications 11 à 13, caractérisé en ce que, par rapport à la quantité de chitosane utilisée, on utilise l'oxyde de butylène trois à quinze fois en excès.